# EUROPEAN PATENT APPLICATION

(11) **EP 4 640 225 A1**
(43) Date of publication of application: **29.10.2025**
(21) Application number: 23907003.0
(22) Date of filing: 19.12.2023
(51) Int. Cl.: A61K 35/17, A61K 31/7088, A61K 35/12, A61K 35/76, A61K 38/08, A61K 39/00, A61K 48/00, A61P 35/00, A61P 43/00, C07K 7/06, C07K 14/725, C12N 5/10, C12N 15/12, C12N 15/63

(54) **PHARMACEUTICAL COMPOSITION FOR TREATING OR PREVENTING CANCER**

(30) Priority: 20.12.2022 JP 2022203215
(71) Applicant: NEC Corporation, 108-8001 Tokyo (JP); Aichi Prefecture, Nagoya-shi Aichi 460-0001 (JP)
(72) Inventor: ONOGUCHI Kazuhide, Tokyo 108-8001 (JP); YOSHIHARA Yoshiko, Tokyo 108-8001 (JP); ONOUE Kousuke, Tokyo 108-8001 (JP); TANAKA Yuki, Tokyo 108-8001 (JP); MATSUSHITA Hirokazu, Nagoya-shi, Aichi 464-8681 (JP); MURAOKA Daisuke, Nagoya-shi, Aichi 464-8681 (JP); OKAMURA Ayako, Nagoya-shi, Aichi 464-8681 (JP)
(74) Representative: Betten & Resch
(86) International application number: PCT/JP2023/045398
(87) International publication number: WO 2024/135643

(57) **Abstract**

As a technology available to immunotherapy against cancer expressing KitaKyushu Lung Cancer Antigen 1 (KK-LC-1), there is provided a pharmaceutical composition for administering a peptide, a polynucleotide encoding the peptide, or a vector containing the polynucleotide, in combination with a T cell for treating or preventing cancer expressing KK-LC-1 in a subject, in which the peptide and the T cell have combination (1) or (2) depending on a human leukocyte antigen (HLA) type that the subject has: (1) a combination of a peptide comprising the amino acid sequence of SEQ ID NO: 1 and a T cell expressing a T cell receptor (TCR) having a β-chain comprising complementarity determining region 3 (CDR3) of SEQ ID NO: 2, when the HLA type of the subject is HLA*C14, or (2) a combination of a peptide comprising the amino acid sequence of SEQ ID NO: 3 and a T cell expressing a TCR having a β-chain comprising CDR3 of SEQ ID NO: 4, 5 or 6, when the HLA type of the subject is HLA*B15.

## Description

This application is based upon and claims the benefit of priority from Japanese patent application No. 2022-203215, filed on December 20, 2022, the disclosure of which is incorporated herein in its entirety by reference.

### Technical Field

The present disclosure relates to a pharmaceutical composition for treating or preventing cancer. More specifically, the present invention relates to a pharmaceutical composition for administering, e.g., a peptide in combination with a T cell for treating or preventing cancer expressing KitaKyushu Lung Cancer Antigen 1 (KK-LC-1).

### Background Art

KitaKyushu Lung Cancer Antigen 1 (KK-LC-1) is a cancer antigen that is expressed in various types of cancers such as lung cancer, stomach cancer, breast cancer, and liver cancer. Of normal tissues, KK-LC-1 is expressed only in the testis. Patent Literature 1 discloses a reagent for cancer immunotherapy using a peptide derived from KK-LC-1.

### Citation List

### Patent Literature

Patent Literature 1: WO 2017/089756 A

### Summary of Invention

### Technical Problem

It is a main object of the present disclosure to provide techniques available for immunotherapy against cancers expressing KK-LC-1.

### Solution to Problem

In order to solve the above problems, the present disclosure provides the following [1] to [23].
[1] A pharmaceutical composition
   for administering a peptide, a polynucleotide encoding the peptide, or a vector containing the polynucleotide, in combination with
   a T cell,
   for treating or preventing cancer expressing KK-LC-1 in a subject, in which
   the peptide and the T cell have the following combination (1) or (2) depending on the human leukocyte antigen (HLA) type that the subject has:
      (1) a combination of a peptide comprising the amino acid sequence of SEQ ID NO: 1 and a T cell expressing a T cell receptor (TCR) having a β-chain comprising complementarity determining region 3 (CDR3) of SEQ ID NO: 2, when the HLA type of the subject is HLA*C14,
      (2) a combination of a peptide comprising the amino acid sequence of SEQ ID NO: 3 and a T cell expressing a TCR having a β-chain comprising CDR3 of SEQ ID NO: 4, 5 or 6 when the HLA type of the subject is HLA*B15.
[2] A pharmaceutical composition for administering
   a peptide, a polynucleotide encoding the peptide, or a vector containing the polynucleotide, in combination with
   a T cell,
   for treating or preventing cancer expressing KK-LC-1 in a subject, in which
   the peptide and the T cell have a combination of the following (1) or (2):
      (1) a combination of a peptide comprising the amino acid sequence of SEQ ID NO: 1 and a T cell expressing a TCR having a β-chain comprising CDR3 of SEQ ID NO: 2,
      (2) a combination of a peptide comprising the amino acid sequence of SEQ ID NO: 3 and a T cell expressing a TCR having a β-chain comprising CDR3 of SEQ ID NO: 4, 5 or 6.
[3] The pharmaceutical composition according to [2], in which
   an HLA type that the subject has is
   HLA*C14 in the combination (1), and
   HLA*B15 in the combination (2).
[4] The pharmaceutical composition according to [3], in which
   the HLA type that the subject has is
   HLA*C14:02 in the combination (1), and
   HLA*B15:01 in the combination (2).
[5] The pharmaceutical composition according to any one of [1] to [4], in which the cancer is lung cancer, stomach cancer, breast cancer, or liver cancer.
[6] A pharmaceutical composition comprising a peptide, a polynucleotide encoding the peptide or a vector containing the polynucleotide, which is administered in combination with a T cell, for treating or preventing cancer expressing KK-LC-1 in a subject, in which
   the peptide is the following (1) or (2):
   (1) a peptide comprising the amino acid sequence of SEQ ID NO: 1, which is administered in combination with a T cell expressing a TCR having a β-chain comprising CDR3 of SEQ ID NO: 2,
   (2) a peptide comprising the amino acid sequence of SEQ ID NO: 3, which is administered in combination with a T cell expressing a TCR having a β-chain comprising CDR3 of SEQ ID NO: 4, 5 or 6.
[7] A pharmaceutical composition for treating or preventing cancer expressing KK-LC-1 in a subject, comprising a peptide, a polynucleotide encoding the peptide, or a vector containing the polynucleotide, in which
   the peptide is the following (1) or (2):
   (1) a peptide comprising the amino acid sequence of SEQ ID NO: 1,
   (2) a peptide comprising the amino acid sequence of SEQ ID NO: 3.
[8] The pharmaceutical composition of [6] or [7], which is a cancer vaccine.
[9] The pharmaceutical composition according to any one of [6] to [8], in which the cancer is lung cancer, stomach cancer, breast cancer, or liver cancer.
[10] A peptide comprising the amino acid sequence of SEQ ID NO: 1 or 3, a polynucleotide encoding the peptide, or a vector containing the polynucleotide.
[11] A pharmaceutical composition comprising a T cell, which is administered in combination with a peptide, a polynucleotide encoding the peptide, or a vector containing the polynucleotide, for treating or preventing cancer expressing KK-LC-1 in a subject, in which
   the T cell is the following (1) or (2):
   (1) a T cell expressing a TCR having a β-chain comprising CDR3 of SEQ ID NO: 2, which is administered in combination with a peptide comprising the amino acid sequence of SEQ ID NO: 1,
   (2) a T cell expressing a TCR having a β-chain comprising CDR3 of SEQ ID NO: 4, 5 or 6, which is administered in combination with a peptide comprising the amino acid sequence of SEQ ID NO: 3.
[12] A pharmaceutical composition for treating or preventing cancer expressing KK-LC-1 in a subject, comprising a T cell, in which
   the T cell is the following (1) or (2):
   (1) a T cell expressing a TCR having a β-chain comprising CDR3 of SEQ ID NO: 2,
   (2) a T cell expressing a TCR having a β-chain comprising CDR3 of SEQ ID NO: 4, 5 or 6.
[13] The pharmaceutical composition of [11] or [12], in which the cancer is lung cancer, stomach cancer, breast cancer, or liver cancer.
[14] A polynucleotide encoding a TCR having a β-chain comprising CDR3 of SEQ ID NO: 2, 4, 5 or 6, or a vector containing the polynucleotide.
[15] A method for treating or preventing cancer expressing KK-LC-1 in a subject, including
   a step of administering
   a peptide, a polynucleotide encoding the peptide, or a vector containing the polynucleotide, in combination with a T cell,
   to the subject, in which
   the peptide and the T cell have a combination of the following (1) or (2) depending on the human leukocyte antigen (HLA) type that the subject has:
      (1) a combination of a peptide comprising the amino acid sequence of SEQ ID NO: 1 and a T cell expressing a TCR having a β-chain comprising CDR3 of SEQ ID NO: 2,
      (2) a combination of a peptide comprising the amino acid sequence of SEQ ID NO: 3 and a T cell expressing a TCR having a β-chain comprising CDR3 of SEQ ID NO: 4, 5 or 6.
[16] A method for treating or preventing cancer expressing KK-LC-1 in a subject, including
   a step of determining the HLA type of the subject, and
   a step of administering a peptide, a polynucleotide encoding the peptide, or a vector containing the polynucleotide in combination with
   a T cell, in which
      (1) administering a peptide comprising the amino acid sequence of SEQ ID NO: 1 in combination with a T cell expressing a TCR having a β-chain comprising CDR3 of SEQ ID NO: 2, when the HLA type of the subject is HLA*C14, and
      (2) administering a peptide comprising the amino acid sequence of SEQ ID NO: 3 in combination with a T cell expressing a TCR having a β-chain comprising CDR3 of SEQ ID NO: 4, 5 or 6, when the HLA type of the subject is HLA*B15.
[17] The method according to [15] or [16], in which
   the HLA type that the subject has is
   HLA*C14:02 in the step (1), and
   HLA*B15:01 in the step (2).
[18] The method according to any one of [15] to [17], in which the cancer is lung cancer, stomach cancer, breast cancer, or liver cancer.
[19] A method for treating or preventing cancer expressing KK-LC-1 in a subject, including
   a step of administering a peptide, a polynucleotide encoding the peptide, or a vector containing the polynucleotide to the subject, in which
   the peptide is the following (1) or (2):
      (1) a peptide comprising the amino acid sequence of SEQ ID NO: 1,
      (2) a peptide comprising the amino acid sequence of SEQ ID NO: 3.
[20] A method for treating or preventing cancer expressing KK-LC-1 in a subject, including:
   a step of determining the HLA type of the subject,
      and
   a step of administering a peptide, a polynucleotide encoding the peptide, or a vector containing the polynucleotide to the subject, in which
   the peptide is the following (1) or (2):
      (1) a peptide comprising the amino acid sequence of SEQ ID NO: 1 when the HLA type of the subject is HLA*C14,
      (2) a peptide comprising the amino acid sequence of SEQ ID NO: 3 when the HLA type of the subject is HLA*B15.
[21] A method for treating or preventing cancer expressing KK-LC-1 in a subject, including:
   a step of administering a T cell to the subject, in which
   the T cell is the following (1) or (2):
      (1) a T cell expressing a TCR having a β-chain comprising CDR3 of SEQ ID NO: 2,
      (2) a T cell expressing a TCR having a β-chain comprising CDR3 of SEQ ID NO: 4, 5 or 6.
[22] A method for treating or preventing cancer expressing KK-LC-1 in a subject, including
   a step of determining the HLA type of the subject,
      and
   a step of administering a T cell to the subject, in which
   the T cell is the following (1) or (2):
      (1) a T cell expressing a TCR having a β-chain comprising CDR3 of SEQ ID NO: 2, when the HLA type of the subject is HLA*C14,
      (2) a T cell expressing a TCR having a β-chain comprising CDR3 of SEQ ID NO: 4, 5 or 6, when the HLA type of the subject is HLA*B15.
[23] Use of a peptide, a polynucleotide encoding the peptide, or a vector containing the polynucleotide, or
   a T cell
   for producing a pharmaceutical composition for treating or preventing cancer expressing KK-LC-1 in a subject, in which
   the pharmaceutical composition has a combination of the peptide of the following (1) or (2) and the T cell:
      (1) a combination of a peptide comprising the amino acid sequence of SEQ ID NO: 1 and a T cell expressing a T cell receptor (TCR) having a β-chain comprising CDR3 of SEQ ID NO: 2,
      (2) a combination of a peptide comprising the amino acid sequence of SEQ ID NO: 3 and a T cell expressing a TCR having a β-chain comprising CDR3 of SEQ ID NO: 4, 5 or 6.

In the present disclosure, "immunogenicity" refers to an ability (immunity induction) to induce an immune reaction, and for example, means enhancing the cytotoxic T cell (CTL) inducing activity of the antigen-presenting cell and further enhancing the cytotoxic activity of the CTL against a cancer cell.

In the present disclosure, "CTL induction" means that *in vitro* or *in vivo,* a peptide according to the present disclosure is presented on the surface of an antigen-presenting cell to thereby induce or proliferate a CTL specifically recognizing a KK-LC-1 antigen, or differentiate a naive T cell into an effector cell having the ability (cytotoxic activity) to kill a target cell such as a cancer cell, and/or enhance the cytotoxic activity of the CTL.

The CTL induction activity can be measured by evaluating the production of cytokines (e.g., IFN-γ) by CTLs. For example, CTL inducing activity may be measured by evaluating an increase of cytokine-producing cells induced from precursor cells by an antigen-presenting cell such as a peripheral blood mononuclear cell stimulated with the peptide according to the present disclosure by use of a commonly known highly sensitive immunoassay such as an ELISPOT (Enzyme-LinkedImmunoSpot) or an ELISA (Enzyme-Linked ImmunoSorbent Assay).

The cytotoxic activity of a CTL can also be measured by a known method such as a ⁵¹Cr release assay.

When the CTL inducing activity of a test substance is significantly high compared to a control substance, for example, higher by 5% or more, 10% or more, 20% or more, 30% or more, 40% or more, preferably 50% or more, it can be determined that the test substance has CTL inducing activity.

### Advantageous Effects of Invention

The present disclosure provides techniques available for immunotherapy against cancers that express KK-LC-1.

### Brief Description of Drawings

Fig. 1 shows the detection results of an antigen-specific reaction in co-culture of B-APC loaded with KK-LC-1 peptide and TexTCR-expressing cells expressing TexTCR (pt. 2-ex08TCR).
Fig. 2 shows the detection results of an antigen-specific reaction in co-culture of an HLA expressing cell loaded with the peptide CT83-12 (RFQRNTGEM) and a TexTCR expressing cell expressing TexTCR (pt. 2-ex08TCR).
Fig. 3 shows the detection results of an antigen-specific reaction in co-culture of B-APC loaded with KK-LC-1 peptide and TexTCR-expressing cells expressing TexTCR (pt. 1-ex08TCR, pt. 1-ex15TCR or pt. 2-ex09TCR).
Fig. 4 shows the amino acid sequences of the full-lengths of the β-chain and the α chain and CDRs of TexTCR (pt. 2-ex08TCR).
Fig. 5 shows the amino acid sequences of full-lengths of the β-chain and the α chain and CDRs of TexTCR (pt. 1-ex08TCR) .
Fig. 6 shows the amino acid sequences of full-lengths of the β-chain and the α chain and CDRs of TexTCR (pt. 1-ex15TCR) .
Fig. 7 shows the amino acid sequences of full-lengths of the β-chain and the α chain and CDRs of TexTCR (pt. 2-ex09TCR).

### Description of Embodiments

### [Peptide]

The peptide according to the present disclosure contains the amino acid sequence of SEQ ID NO: 1 or 3.

The peptide according to the present disclosure is presented by HLA of an antigen-presenting cell and recognized by TCR of a T cell. Thus, the peptide according to the present disclosure can be effectively used for immunotherapy against cancer expressing KK-LC-1.

The amino acid sequence of SEQ ID NO: 1 corresponds to positions 23 to 31 from the N-terminus of the KK-LC-1 full-length amino acid sequence. The peptide comprising the amino acid sequence of SEQ ID NO: 1 has HLA*C14 restriction, particularly HLA*C14:02 restriction. Thus, the peptide comprising the amino acid sequence of SEQ ID NO: 1 can be suitably used for CTL induction in a subject having an HLA type of HLA*C14, particularly HLA*C14:02.

The amino acid sequence of SEQ ID NO: 3 corresponds to positions 76 to 84 from the N-terminus of the KK-LC-1 full-length amino acid sequence. The peptide comprising the amino acid sequence of SEQ ID NO: 3 has HLA*B15: restriction in particular HLA*B15:01 restriction. Thus, the peptide comprising the amino acid sequence of SEQ ID NO: 3 may be suitably used for CTL induction in a subject having an HLA type of HLA*B15, particularly HLA*B15:01.

The peptide according to the present disclosure may contain a full-length or part of the amino acid sequence of SEQ ID NO: 1 or 3, and the number of full-length amino acids thereof is preferably 8 to 11 for presentation by HLA.

The peptide according to the present disclosure preferably comprises the full-length amino acid sequence of SEQ ID NO: 1 or 3, and particularly preferably consists of the amino acid sequence of SEQ ID NO: 1 or 3.

In the peptide according to the present disclosure, amino acid residues constituting an amino acid sequence or a part thereof may be modified. Examples of the modification include, but are not particularly limited to, sugar-chain addition, side-chain oxidation, and phosphorylation.

The peptide according to the present disclosure may have substitution, insertion, deletion, or addition of one or several amino acid residues as long as the peptide retains immunogenicity. When the peptide is directly administered into the body, the terminal of the peptide is sometimes changed by, e.g., digestion, for example, in the digestive organ depending on the administration route. Thus, the peptide according to the present disclosure may be present in the form of a precursor having one or more amino acid residues added at the N-terminus and/or the C-terminus thereof.

The peptide according to the present disclosure can be substituted by a polynucleotide encoding the peptide, a vector containing the polynucleotide, or an antigen-presenting cell presenting a complex of the peptide and an HLA molecule on the surface. The peptide according to the present disclosure induces CTLs by being presented by HLA of an antigen-presenting cell, and induced CTLs damage cancer cells. However, CTLs can be induced by administration of a polynucleotide, vector, and antigen-presenting cell thereof in the same manner as in administration of the peptide.

### [T cell]

The T cell according to the present disclosure expresses a TCR having a β-chain comprising CDR3 of any one of SEQ ID NOs: 2 and 4 to 6.

In the binding between a complex of an HLA molecule and an antigen peptide presented by the HLA molecule and a TCR, CDR3 is involved in the recognition of the antigen peptide, and the binding to the antigen peptide is particularly determined by CDR3 of the β-chain.

The TCR of the T cell according to the present disclosure recognizes a KK-LC-1 peptide presented by HLA. Thus, the T cell according to the present disclosure can be effectively used for immunotherapy against cancer expressing KK-LC-1.

The T cell expressing a TCR having a β-chain comprising CDR3 of SEQ ID NO: 2 can bind to an HLA molecule presenting a peptide comprising the amino acid sequence of SEQ ID NO: 1. The peptide comprising the amino acid sequence of SEQ ID NO: 1 has HLA*C14 (particularly HLA*C14:02) restriction. Thus, the T cell expressing a TCR having a β-chain comprising CDR3 of SEQ ID NO: 2 is preferably to be administered in combination with a peptide comprising the amino acid sequence of SEQ ID NO: 1, and more preferably administered together with the peptide to a subject having an HLA type of HLA*C14 (particularly HLA*C14:02).

An example of the T cell expressing the TCR having the β-chain comprising CDR3 of SEQ ID NO: 2 is a T cell comprising an HLA binding region (CDR1) of SEQ ID NO: 17 and an HLA binding region (CDR2) of SEQ ID NO: 18 as complementarity determining regions of the β-chain, and further comprising an HLA binding region (CDR1) of SEQ ID NO: 19, an HLA binding region (CDR2) of SEQ ID NO: 20 and an HLA binding region (CDR3) of SEQ ID NO: 21 as complementarity determining regions of the α chain.

A T cell expressing a TCR having a β-chain comprising CDR3 of SEQ ID NO: 2 may contain the amino acid sequence of SEQ ID NO: 22 as the full-length sequence of the β-chain and the amino acid sequence of SEQ ID NO: 23 as the full-length sequence of the α-chain.

A T cell expressing a TCR having a β-chain comprising CDR3 of SEQ ID NO: 4, 5 or 6 may bind to an HLA molecule presenting a peptide comprising the amino acid sequence of SEQ ID NO: 3. The peptide comprising the amino acid sequence of SEQ ID NO: 3 has HLA-B15 (particularly HLA-B15:01) restriction. Thus, the T cell expressing a TCR having a β-chain comprising CDR3 of SEQ ID NO: 4, 5 or 6 is preferably to be administered in combination with a peptide comprising the amino acid sequence of SEQ ID NO: 3, and more preferably administered together with the peptide to a subject having an HLA type of HLA-B15 (particularly HLA-B15:01).

An example of the T cell expressing the TCR having the β-chain comprising CDR3 of SEQ ID NO: 4 is a T cell comprising an HLA binding region (CDR1) of SEQ ID NO: 24 and an HLA binding region (CDR2) of SEQ ID NO: 25 as complementarity determining regions of the β-chain, and further comprising an HLA binding region (CDR1) of SEQ ID NO: 26, an HLA binding region (CDR2) of SEQ ID NO: 27 and an HLA binding region (CDR3) of SEQ ID NO: 28 as complementarity determining regions of the α chain.

The T cell expressing the TCR having the β-chain comprising CDR3 of SEQ ID NO: 4 may contain the amino acid sequence of SEQ ID NO: 29 as the full-length sequence of the β-chain and the amino acid sequence of SEQ ID NO: 30 as the full-length sequence of the α chain.

An example, the T cell expressing the TCR having the β-chain comprising CDR3 of SEQ ID NO: 5 is a T cell comprising an HLA binding region (CDR1) of SEQ ID NO: 31 and an HLA binding region (CDR2) of SEQ ID NO: 32 as complementarity determining regions of the β-chain and further comprising an HLA binding region (CDR1) of SEQ ID NO: 33, an HLA binding region (CDR2) of SEQ ID NO: 34 and an HLA binding region (CDR3) of SEQ ID NO: 35 as complementarity determining regions of the α chain.

The T cell expressing the TCR having the β-chain comprising CDR3 of SEQ ID NO: 5 may contain the amino acid sequence of SEQ ID NO: 36 as the full-length sequence of the β-chain and the amino acid sequence of SEQ ID NO: 37 as the full-length sequence of the α chain.

An example of the T cell expressing the TCR having the β-chain comprising CDR3 of SEQ ID NO: 6 is a T cell comprising an HLA binding region (CDR1) of SEQ ID NO: 38 and an HLA binding region (CDR2) of SEQ ID NO: 39 as complementarity determining regions of the β-chain, and further comprising an HLA binding region (CDR1) of SEQ ID NO: 40, an HLA binding region (CDR2) of SEQ ID NO: 41 and an HLA binding region (CDR3) of SEQ ID NO: 42 as complementarity determining regions of the α chain.

The T cell expressing the TCR having the β-chain comprising CDR3 of SEQ ID NO: 6 may contain the amino acid sequence of SEQ ID NO: 43 as the full-length sequence of the β-chain and the amino acid sequence of SEQ ID NO: 44 as the full-length sequence of the α chain.

The T cell according to the present disclosure may be a T cell (TCR gene-introduced T cell) to be expressed by introduction of a gene of the aforementioned TCR.

A peripheral blood lymphocyte derived from a subject in need of treatment or prevention of cancer is transformed with a vector containing a polynucleotide encoding a TCR, and the resulting T cell having a TCR gene introduced therein is proliferated and then returned to the body of the subject.

A polynucleotide encoding a TCR or a vector containing the polynucleotide may be introduced into the peripheral blood lymphocyte by a conventionally known method such as a lipofection method, an electroporation method, a microinjection method, a cell fusion method, a DEAE dextran method, or a calcium phosphate method.

The T cell according to the present disclosure can be replaced by a T cell (CAR-T) expressing a CAR comprising an antigen recognition region derived from an antibody that binds to a complex of a peptide according to the present disclosure and an HLA molecule. As the antigen recognition region, scFv is generally used.

### [Pharmaceutical Composition]

The pharmaceutical composition according to the present disclosure is a pharmaceutical composition for administering the aforementioned peptide, a polynucleotide encoding the peptide, or a vector containing the polynucleotide, in combination with a T cell, in which the peptide and the T cell have a combination of the following (1) or (2):
(1) a combination of a peptide comprising the amino acid sequence of SEQ ID NO: 1 and a T cell expressing a TCR having a β-chain comprising CDR3 of SEQ ID NO: 2,
(2) a combination of a peptide comprising the amino acid sequence of SEQ ID NO: 3 and a T cell expressing a TCR having a β-chain comprising CDR3 of SEQ ID NO: 4, 5 or 6.

In another embodiment, the pharmaceutical composition according to the present disclosure may contain only the peptide described in the above (1) or (2) alone or a T cell alone.

The peptide comprising the amino acid sequence of SEQ ID NO: 1 is presented by an HLA molecule of HLA*C14 (in particular HLA*C14:02) and recognized by a T cell expressing a TCR having a β-chain comprising CDR3 of SEQ ID NO: 2. Thus, the peptide comprising the amino acid sequence of SEQ ID NO: 1 and the T cell expressing the TCR having a β-chain comprising CDR3 of SEQ ID NO: 2 can be administered in combination to a subject having an HLA type of HLA*C14 (particularly HLA*C14:02). In this manner, the peptide can be particularly effectively used for treating or preventing cancer expressing KK-LC-1.

The peptide comprising the amino acid sequence of SEQ ID NO: 3 is presented by an HLA molecule of HLA-B15 (in particular HLA-B15:01) and recognized by a T cell expressing a TCR having a β-chain comprising CDR3 of SEQ ID NO: 4, 5 or 6. Thus, the peptide comprising the amino acid sequence of SEQ ID NO: 3 and the T cell expressing the TCR having the β-chain comprising CDR3 of SEQ ID NO: 4, 5 or 6 can be administered in combination to a subject having an HLA type of HLA-B15 (particularly HLA-B15:01). In this manner, the peptide can be particularly effectively used for treating or preventing cancer expressing KK-LC-1.

The cancer to which the pharmaceutical composition according to the present disclosure can be applied is not particularly limited as long as it is a cancer expressing KK-LC-1. The cancer may be, for example, lung cancer, stomach cancer, breast cancer, or liver cancer.

The pharmaceutical composition according to the present disclosure can be used not only for treating cancer but also preventing cancer. For example, when the pharmaceutical composition according to the present disclosure is administered to a healthy human body, CTLs are induced and the induced CTLs remain in the body, with the result that cancer cells if develop can be damaged. Similarly, recurrence of cancer may be prevented by administering the pharmaceutical composition to the human body after completion of cancer treatment.

In the pharmaceutical composition according to the present disclosure, the peptide and a T cell may be contained in the same or separate preparations. When the peptide and the T cell are contained in separate compositions, the preparation comprising the peptide and the preparation comprising the T cell may be administered to the subject at the same time or at different times.

The dose of the peptide and the T cell can be appropriately set depending on, e.g., the age and weight of the patient and the administration method so as to obtain the intended immunogenicity.

The pharmaceutical composition according to the present disclosure may contain other components known to be used for cancer therapy, for example, chemokines, cytokines, tumor necrosis factors, and chemotherapeutic agents.

The pharmaceutical composition according to the present disclosure may contain various additives. Examples of the additive include a preservative and a buffer. Examples of the preservative include sodium bisulfite, sodium bisulfite, sodium benzalkonium thiosulfate chloride, chlorobutanol, thimerosal, phenylmercuric acetate, phenylmercuric nitrate, methylparaben, polyvinyl alcohol, phenylethyl alcohol, ammonia, dithiothreitol, and betamercaptoethanol. Examples of the buffer include sodium carbonate, sodium borate, sodium phosphate, sodium acetate, and sodium bicarbonate.

The pharmaceutical composition according to the present disclosure can be used as a cancer vaccine in an embodiment comprising a peptide and not comprising a T cell.

In addition, the vaccine may contain an inactive component for enhancing the effect as a vaccine. Examples of the inactive component include an adjuvants and a toxoid. Examples of the adjuvant include a precipitation type of adjuvant such as aluminum hydroxide, aluminum phosphate, and calcium phosphate and an oily type of adjuvant such as Freund's complete adjuvant and Freund's incomplete adjuvant.

In an embodiment of the pharmaceutical composition according to the present disclosure comprising a peptide and not comprising a T cell, the pharmaceutical composition is intended not only for administration to the human body but also for use outside the body.

More specifically, the pharmaceutical composition according to the present disclosure may be used to stimulate an antigen-presenting cell *in vitro* or *ex vivo* to enhance CTL induction activity. For example, the pharmaceutical composition according to the present disclosure is brought into contact with an antigen-presenting cell, such as a dendritic cell, derived from a subject in need of treatment or prevention of cancer, and then the antigen-presenting cell is returned into the body of the subject.

The peptide may be introduced into the antigen-presenting cell by, for example, a lipofection method or an injection method. In addition, a polynucleotide encoding the peptide or a vector containing the polynucleotide may be introduced into an antigen-presenting cell by, for example, a lipofection method, an electroporation method, a microinjection method, a cell fusion method, a DEAE dextran method or a calcium phosphate method to express the peptide.

A vector refers to a nucleic acid construct that enables expression of an mRNA, a protein or a polypeptide in a host cell. As the vector, for example, a known vector such as a plasmid or a virus may be used.

### [Therapeutic/Preventive Method and Companion Diagnosis]

The method for treating or preventing cancer according to the present disclosure includes a step of administering the aforementioned peptide, a polynucleotide encoding the peptide, or a vector containing the polynucleotide in combination with a T cell, in which
the peptide and the T cell have a combination of the following (1) or (2):
(1) a combination of a peptide comprising the amino acid sequence of SEQ ID NO: 1 and a T cell expressing a TCR having a β-chain comprising CDR3 of SEQ ID NO: 2,
(2) a combination of a peptide comprising the amino acid sequence of SEQ ID NO: 3 and a T cell expressing a TCR having a β-chain comprising CDR3 of SEQ ID NO: 4, 5 or 6.

The peptide, the polynucleotide encoding the peptide, or the vector containing the polynucleotide (hereinafter, also sometimes collectively referred to simply as "peptide") and the T cell may be administered as the same preparation, or separate preparations. The administration of the peptide and the administration of the T cell may be performed simultaneously or separately. The administration routes of peptide and the T cell may be the same or different.

In another embodiment, the method for treating or preventing cancer according to the present disclosure may include a step of administering the peptide described in the above (1) or (2), a polynucleotide encoding the peptide, or a vector containing the polynucleotide alone, or a T cell alone.

The method for treating or preventing cancer according to the present disclosure may include a step for determining an HLA genotype of a subject before the step of administering a peptide and/or a T cell. The HLA genotype can be determined using a conventionally known genetic analysis method.

The peptide comprising the amino acid sequence of SEQ ID NO: 1 and the T cell expressing the TCR having the β-chain comprising CDR3 of SEQ ID NO: 2 can be expected to produce an optimized therapeutic effect by being administered to a subject having an HLA type of HLA*C14 (particularly HLA*C14:02).

In addition, the peptide comprising the amino acid sequence of SEQ ID NO: 3 and the T cell expressing the TCR having the β-chain comprising CDR3 of SEQ ID NO: 4, 5 or 6 can be expected to produce an optimized therapeutic effect by being administered to a subject particularly having an HLA type of HLA-B15 (particularly HLA-B15:01).

Thus, a high therapeutic effect can be expected by determining the HLA genotype of the subject before the administration step of the peptide and/or the T cell, and selecting the peptide, the T cell, or a combination thereof in accordance with the HLA type of the subject.

A whole or part of the above embodiments can also be described as follows, but are not limited to the followings. Note that, in the constitution of the invention described below, the constitution according to one category of the invention can also be subordinated to another category of the invention.
[1] A pharmaceutical composition
   for administering a peptide, a polynucleotide encoding the peptide, or a vector containing the polynucleotide, in combination with
   a T cell,
   for treating or preventing cancer expressing KK-LC-1 in a subject, in which
   the peptide and the T cell have the following combination (1) or (2) depending on the human leukocyte antigen (HLA) type that the subject has:
      (1) a combination of a peptide comprising the amino acid sequence of SEQ ID NO: 1 and a T cell expressing a T cell receptor (TCR) having a β-chain comprising complementarity determining region 3 (CDR3) of SEQ ID NO: 2, when the HLA type of the subject is HLA*C14,
      (2) a combination of a peptide comprising the amino acid sequence of SEQ ID NO: 3 and a T cell expressing a TCR having a β-chain comprising CDR3 of SEQ ID NO: 4, 5 or 6 when the HLA type of the subject is HLA*B15.
[2] A pharmaceutical composition for administering
   a peptide, a polynucleotide encoding the peptide, or a vector containing the polynucleotide, in combination with
   a T cell,
   for treating or preventing cancer expressing KK-LC-1 in a subject, in which
   the peptide and the T cell have a combination of the following (1) or (2):
      (1) a combination of a peptide comprising the amino acid sequence of SEQ ID NO: 1 and a T cell expressing a TCR having a β-chain comprising CDR3 of SEQ ID NO: 2,
      (2) a combination of a peptide comprising the amino acid sequence of SEQ ID NO: 3 and a T cell expressing a TCR having a β-chain comprising CDR3 of SEQ ID NO: 4, 5 or 6.
[3] The pharmaceutical composition according to [2], in which
   an HLA type that the subject has is
   HLA*C14 in the combination (1), and
   HLA*B15 in the combination (2).
[4] The pharmaceutical composition according to [3], in which
   the HLA type that the subject has is
   HLA*C14:02 in the combination (1), and
   HLA*B15:01 in the combination (2).
[5] The pharmaceutical composition according to any one of [1] to [4], in which the cancer is lung cancer, stomach cancer, breast cancer, or liver cancer.
[6] A pharmaceutical composition comprising a peptide, a polynucleotide encoding the peptide or a vector containing the polynucleotide, which is administered in combination with a T cell for treating or preventing cancer expressing KK-LC-1 in a subject, in which
   the peptide is the following (1) or (2):
   (1) a peptide comprising the amino acid sequence of SEQ ID NO: 1, which is administered in combination with a T cell expressing a TCR having a β-chain comprising CDR3 of SEQ ID NO: 2,
   (2) a peptide comprising the amino acid sequence of SEQ ID NO: 3, which is administered in combination with a T cell expressing a TCR having a β-chain comprising CDR3 of SEQ ID NO: 4, 5 or 6.
[7] A pharmaceutical composition for treating or preventing cancer expressing KK-LC-1 in a subject, comprising a peptide, a polynucleotide encoding the peptide, or a vector comprising the polynucleotide, in which
   the peptide is the following (1) or (2):
   (1) a peptide comprising the amino acid sequence of SEQ ID NO: 1,
   (2) a peptide comprising the amino acid sequence of SEQ ID NO: 3.
[8] The pharmaceutical composition of [6] or [7], which is a cancer vaccine.
[9] The pharmaceutical composition according to any one of [6] to [8], in which the cancer is lung cancer, stomach cancer, breast cancer, or liver cancer.
[10] A peptide comprising the amino acid sequence of SEQ ID NO: 1 or 3, a polynucleotide encoding the peptide, or a vector containing the polynucleotide.
[11] A pharmaceutical composition comprising a T cell, which is administered in combination with a peptide, a polynucleotide encoding the peptide, or a vector containing the polynucleotide, for treating or preventing cancer expressing KK-LC-1 in a subject, in which
   the T cell is the following (1) or (2):
   (1) a T cell expressing a TCR having a β-chain comprising CDR3 of SEQ ID NO: 2, which is administered in combination with a peptide comprising the amino acid sequence of SEQ ID NO: 1,
   (2) a T cell expressing a TCR having a β-chain comprising CDR3 of SEQ ID NO: 4, 5 or 6, which is administered in combination with a peptide comprising the amino acid sequence of SEQ ID NO: 3.
[12] A pharmaceutical composition for treating or preventing cancer expressing KK-LC-1 in a subject, comprising a T cell, in which
   the T cell is the following (1) or (2):
   (1) a T cell expressing a TCR having a β-chain comprising CDR3 of SEQ ID NO: 2,
   (2) a T cell expressing a TCR having a β-chain comprising CDR3 of SEQ ID NO: 4, 5 or 6.
[13] The pharmaceutical composition of [11] or [12], in which the cancer is lung cancer, stomach cancer, breast cancer, or liver cancer.
[14] A polynucleotide encoding a TCR having a β-chain comprising CDR3 of SEQ ID NO: 2, 4, 5 or 6, or a vector containing the polynucleotide.
[15] A method for treating or preventing cancer expressing KK-LC-1 in a subject, including
   a step of administering
   a peptide, a polynucleotide encoding the peptide, or a vector containing the polynucleotide, in combination with a T cell,
   to the subject, in which
   the peptide and the T cell have a combination of the following (1) or (2) depending on the human leukocyte antigen (HLA) type that the subject has:
      (1) a combination of a peptide comprising the amino acid sequence of SEQ ID NO: 1 and a T cell expressing a TCR having a β-chain comprising CDR3 of SEQ ID NO: 2,
      (2) a combination of a peptide comprising the amino acid sequence of SEQ ID NO: 3 and a T cell expressing a TCR having a β-chain comprising CDR3 of SEQ ID NO: 4, 5 or 6.
[16] A method for treating or preventing cancer expressing KK-LC-1 in a subject, including
   a step of determining the HLA type of the subject,
      and
   a step of administering a peptide, a polynucleotide encoding the peptide, or a vector containing the polynucleotide in combination with
   a T cell, in which
      (1) administering a peptide comprising the amino acid sequence of SEQ ID NO: 1 in combination with a T cell expressing a TCR having a β-chain comprising CDR3 of SEQ ID NO: 2, when the HLA type of the subject is HLA*C14, and
      (2) administering a peptide comprising the amino acid sequence of SEQ ID NO: 3 in combination with a T cell expressing a TCR having a β-chain comprising CDR3 of SEQ ID NO: 4, 5 or 6, when the HLA type of the subject is HLA*B15.
[17] The method according to [15] or [16], in which
   the HLA type that the subject has is
   HLA*C14:02 in the step (1), and
   HLA*B15:01 in the step (2).
[18] The method according to any one of [15] to [17], in which the cancer is lung cancer, stomach cancer, breast cancer, or liver cancer.
[19] A method for treating or preventing cancer expressing KK-LC-1 in a subject, including
   a step of administering a peptide, a polynucleotide encoding the peptide, or a vector containing the polynucleotide to the subject, in which
   the peptide is the following (1) or (2):
      (1) a peptide comprising the amino acid sequence of SEQ ID NO: 1,
      (2) a peptide comprising the amino acid sequence of SEQ ID NO: 3.
[20] A method for treating or preventing cancer expressing KK-LC-1 in a subject, including:
   a step of determining the HLA type of the subject,
      and
   a step of administering a peptide, a polynucleotide encoding the peptide, or a vector containing the polynucleotide to the subject, in which
   the peptide is the following (1) or (2):
      (1) a peptide comprising the amino acid sequence of SEQ ID NO: 1 when the HLA type of the subject is HLA*C14,
      (2) a peptide comprising the amino acid sequence of SEQ ID NO: 3 when the HLA type of the subject is HLA*B15.
[21] The method of [20], in which
   an HLA type that the subject has is
   HLA*C14:02 in the (1), and
   HLA*B15:01 in the (2).
[22] A method for treating or preventing cancer expressing KK-LC-1 in a subject, including:
   a step of administering a T cell to the subject, in which
   the T cell is the following (1) or (2):
      (1) a T cell expressing a TCR having a β-chain comprising CDR3 of SEQ ID NO: 2,
      (2) a T cell expressing a TCR having a β-chain comprising CDR3 of SEQ ID NO: 4, 5 or 6.
[23] A method for treating or preventing cancer expressing KK-LC-1 in a subject, including:
   a step of determining the HLA type of the subject,
   a step of administering a T cell to the subject,
   in which the T cell is the following (1) or (2):
      (1) a T cell expressing a TCR having a β-chain comprising CDR3 of SEQ ID NO: 2, when the HLA type of the subject is HLA*C14,
      (2) a T cell expressing a TCR having a β-chain comprising CDR3 of SEQ ID NO: 4, 5 or 6, when the HLA type of the subject is HLA*B15.
[24] The method of [23], in which
   an HLA type that the subject has is
   HLA*C14:02 in the (1), and
   HLA*B15:01 in the (2).
[24] The method according to [19] to [23], in which the cancer is lung cancer, stomach cancer, breast cancer, or liver cancer.

### Examples

### 1. Preparation of HLA Gene Expression Vector

HLA genes of HLA types A*24:02, A*26:03, B*15:01, B*51:01, C*07:02, and C*14:02 were cloned into expression vectors (pcDNA3.1(+)) to prepare HLA gene expression vectors pcDNA3.1(+)/HLA-A*24:02, pcDNA3.1(+)/HLA-A*26:03, pcDNA3.1(+)/HLA-B*15:01, pcDNA3.1(+)/HLA-B*51:01, pcDNA3.1(+)/HLA-C*07:02, and pcDNA3.1(+)/HLA-C*14:02.

### 2. Introduction of HLA Gene Into HEK293T Cell

To OPTI-MEM medium (250 µL), 5 µL of Lipofectamine 2000 (Thermo Fisher Scientific) was added and allowed to react for 5 minutes. The medium was mixed with OPTI-MEM medium (250 µL) containing an HLA gene expression vector (2.5 µg), and allowed to stand still for 20 minutes. To the mixed liquid, 8 × 10⁵ HEK293T cells were added, and cultured for 6 hours. Exchange of the medium was performed, and culture was performed for further 2 days to obtain HLA expressing cells.

### 3. Introduction of TCR Gene Into Jurkat Cell

A synthetic gene was prepared by ligating cDNA of the β-chain and cDNA of the α-chain of TCR (TexTCR) identified from tumor-infiltrating lymphocytes of a lung cancer patient with a coding sequence of a self-cleaving peptide (P2A). The synthetic gene was inserted into the retroviral vector pMX-IRES-puro (CELL BIOLABS INC) (pMX-IRES-puro/TexTCR) .

A retroviral producer cell was transfected with pMX-IRES-puro/TexTCR (2.5 µg) using Lipofectamine 2000 and cultured. As the retroviral producer cell, a cell obtained by introducing GALV gene into a Phoenix-GP cell (ATCC, CRL-3215) was used.

Six hours after the initiation of the culture, the culture medium was exchanged, and the cell was cultured for further 3 days. The culture supernatant was filtered through a 0.8 um-filter to obtain a virus liquid.

In 1 mL of a retroviral solution, 1 × 10⁵ CD8-expressing Jurkat cells not expressing a TCR were suspended. After polybrene (8 µg/ml) was mixed to the suspension, the suspension was transferred to a 12-well plate, and infection was performed by a spin infection method (1,000 g, one hour, 32°C). After 4 hours, the medium (2 ml/well) was added. Day 2 after the infection, cells were collected and suspended in a fresh medium, and puromycin (1 µg/ml) was added thereto and culture was performed. Two weeks later, the expression of TCR was confirmed by a flow cytometer. CD8-expressing Jurkat cells expressing TexTCR were obtained.

### 4. KK-LC-1 Peptide

KK-LC-1 peptides of the following amino acid sequences were synthesized. A 20 mg/mL solution was prepared using DMSO. The peptide solution was put in use after appropriately diluted.
CT83-S1: ILNNFPHSI (SEQ ID NO: 7)
CT83-S2: RQKRILVNL (SEQ ID NO: 3)
CT83-K1: SILCALIVF (SEQ ID NO: 8)
CT83-K2: IVFWKYRRF (SEQ ID NO: 9)
CT83-K3: LVRPSSSGL (SEQ ID NO: 10)
Ichi010_MP41: YCWEYFLSL (SEQ ID NO: 11)
CT83-7: VRPSSSGLI (SEQ ID NO: 12)
CT83-8: YLLLASSIL (SEQ ID NO: 13)
CT83-9: NFPHSIARQ (SEQ ID NO: 14)
CT83-10: LVELEHTLL (SEQ ID NO: 15)
CT83-11: LLLASSILC (SEQ ID NO: 16)
CT83-12: RFQRNTGEM (SEQ ID NO: 1)

### 5. Detection of Antigen-specific Reaction

TexTCR-expressing cells were genetically modified so as to express luciferase under the control of the TCR signal. More specifically, a solution (2 µg/µL) of a vector (pGL4.30 [luc2P/NFAT-RE/Hygro]) containing a nuclear factor of activated T cells (NFAT) responsive element and a luciferase gene was prepared. The CD8-expressing Jurkat cells expressing TexTCR were washed with PBS and then suspended in a buffer for electroporation. The cell density was controlled so as to obtain 2 × 10⁵ cells/11 µL. After the vector solution (1 µL) and 11 µL of the cell solution were mixed, the mixture was loaded on a Neon Tip, inserted into a Neon Tube containing 3 mL of a buffer, and subjected to electroporation (Thermo Fisher Scientific, Neon transfection system) under the conditions of 1200 V and 5 ms.

After the gene was introduced, the cells were suspended in 100 µL of an antibiotic-free medium.

Target cells were loaded with KK-LC-1 peptide (20 µg/ml) and seeded in a 96-well plate (2 × 10⁴ cells/well). As the target cells, autologous B cell antigen-presenting cells (B-APCs) of a patient were used in addition to the HLA expressing cells prepared in the above section 1. B-APCs were seeded at a density of 5 × 10⁴ cells/well.

CD8-expressing Jurkat cells expressing TexTCR were added to each well (5 x 10⁴ cells/well). After co-culture for 14 hours, a luciferase substrate solution (Promega, Steady-Glo Luciferase Assay System) was added at a concentration of 50 µL/well, and allowed to react at room temperature for 10 minutes. The mixture (70 µL) was transferred to a half-area white plate (Greiner, LUMITRAC 675075) and luminescence was measured by an EnSpire multimode plate reader (PerkinElmer).

### 6. Results

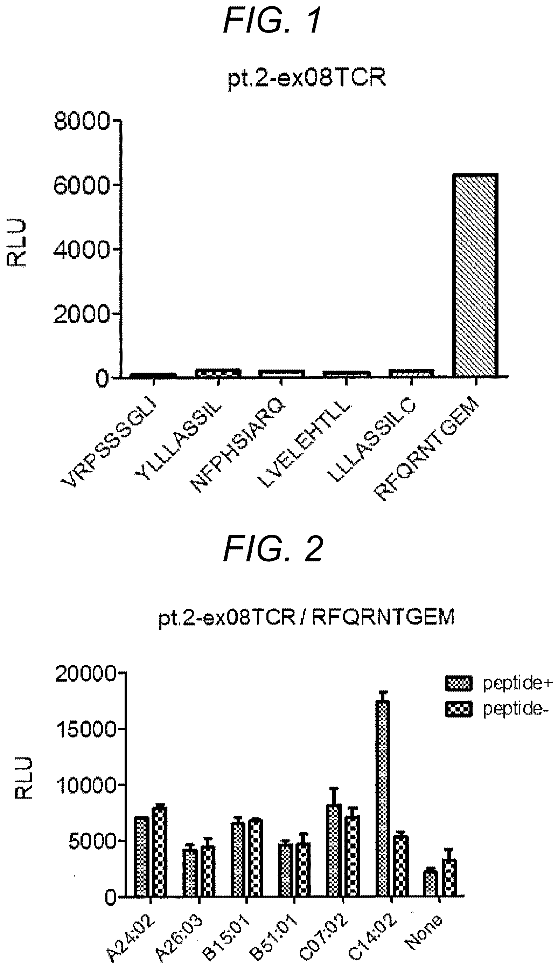

The detection results of the antigen-specific reaction in the co-culture of the B-APC loaded with the peptide and TexTCR-expressing cells in which TexTCR (pt. 2-ex08TCR) was expressed are shown in Fig. 1. An antigen-specific reaction via the peptide CT83-12 (RFQRNTGEM) was detected.

The detection results of the antigen-specific reaction in the co-culture of the HLA expressing cell loaded with the peptide CT83-12 (RFQRNTGEM) and the TexTCR expressing cell in which TexTCR (pt. 2-ex08TCR) was expressed are shown in Fig. 2. It was found that the antigen-specific reaction via peptides CT83-12 (RFQRNTGEM) and TexTCR (pt. 2-ex08TCR) is HLA-C14:02-restrictive. In the figure, "None" indicates the result of an EK293T cell having no introduction of an HLA gene.

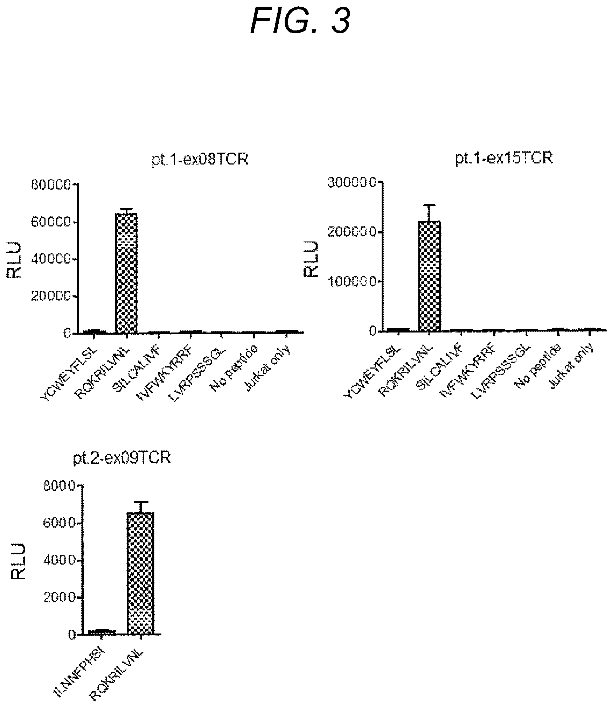

The detection results of the antigen-specific reaction in the co-culture of the B-APC loaded with the peptide and TexTCR-expressing cells in which TexTCR (pt. 1-ex08TCR, pt. 1-ex15TCR, or pt. 2-ex09TCR) was expressed are shown in Fig. 3. Antigen-specific reactions of the peptide CT83-S2 (RQKRILVNL) via all 3 TexTCRs were detected. Note that, the peptide CT83-S2 (RQKRILVNL) is known to be HLA-B15:01-restrictive.

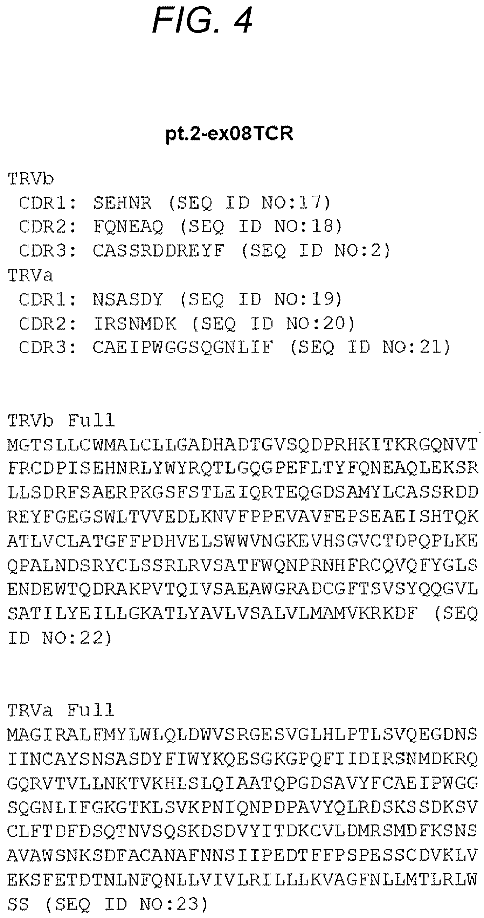

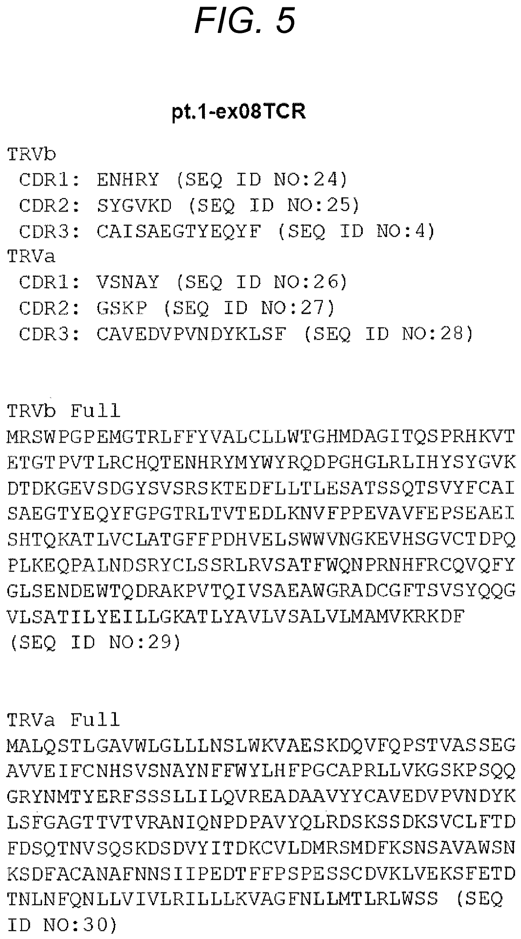

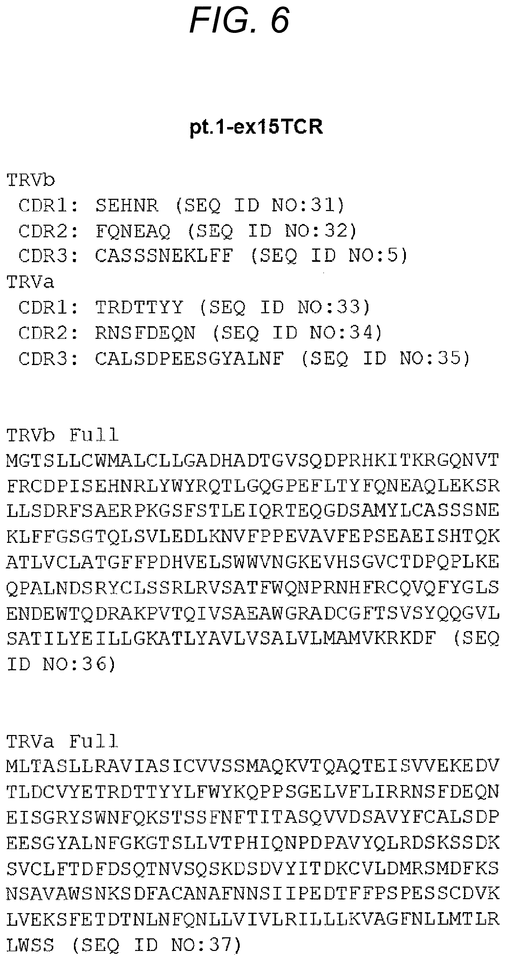

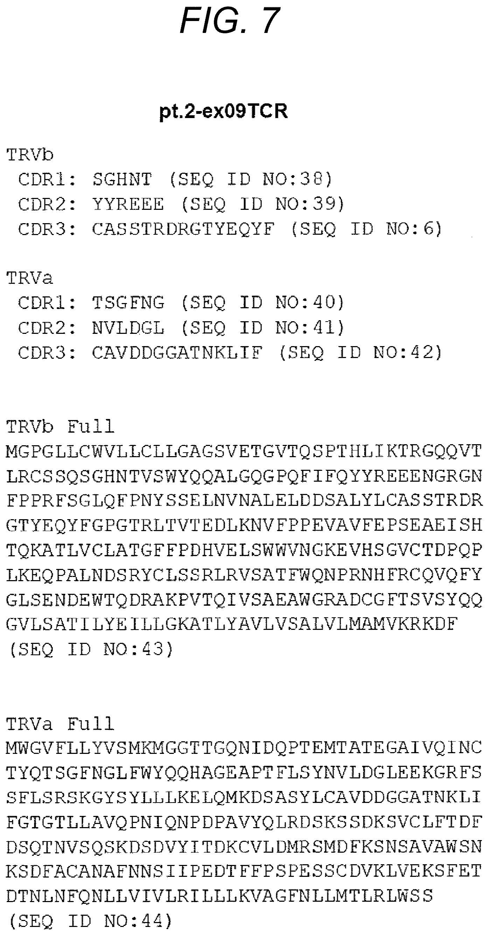

The combinations of HLA types, peptides and TCRs that induced antigen-specific reactions are shown in Table 1. The full-length amino acid sequences and CDR amino acid sequences of the β-chain and the α chain of pt. 2-ex08TCR, pt. 1-ex08TCR, pt. 1-ex15TCR, and pt. 2-ex09TCR are shown in Figs. 4 to 7.

**[Table 1]**

| HLA type | Peptide | TCR |
|---|---|---|
| **HLA-C14:02** | **CT83-12 : RFQRNTGEM** (SEQ ID NO: 1) | **pt.2-ex08TCR** |
| | | β-chain CDR3 (SEQ ID NO: 2) |
| **HLA-B15:01** | **CT83-S2 : RQKRILVNL** (SEQ ID NO: 3) | **pt.1-ex08TCR** |
| | | β-chain CDR3 (SEQ ID NO: 4) |
| | | **pt.1-ex15TCR** |
| | | β-chain CDR3 (SEQ ID NO: 5) |
| | | **pt.2-ex09TCR** |
| | | β-chain CDR3 (SEQ ID NO: 6) |

### Sequence Listing Free Text

SEQ ID NO: 1: Amino acid sequence of peptide CT83-12
SEQ ID NO: 2: Amino acid sequence of β-chain CDR3 of pt. 2-ex08TCR
SEQ ID NO: 3: Amino acid sequence of peptide CT83 to S2
SEQ ID NO: 4: Amino acid sequence of β-chain CDR3 of pt. 1-ex08TCR
SEQ ID NO: 5: Amino acid sequence of β-chain CDR3 of pt. 1-ex15TCR
SEQ ID NO: 6: Amino acid sequence of β-chain CDR3 of pt. 2-ex09TCR
SEQ ID NO: 7: Amino acid sequence of peptide CT83 to S1
SEQ ID NO: 8: Amino acid sequence of peptide CT83-K1
SEQ ID NO: 9: Amino acid sequence of peptide CT83-K2
SEQ ID NO: 10: Amino acid sequence of peptide CT83-K3
SEQ ID NO: 11: Amino acid sequence of peptide Ichi010_MP41
SEQ ID NO: 12: Amino acid sequence of peptide CT83-7
SEQ ID NO: 13: Amino acid sequence of peptide CT83-8
SEQ ID NO: 14: Amino acid sequence of peptide CT83-9
SEQ ID NO: 15: Amino acid sequence of peptide CT83-10
SEQ ID NO: 16: Amino acid sequence of peptide CT83-11
SEQ ID NO: 17: Amino acid sequence of β-chain CDR1 of pt. 2-ex08TCR
SEQ ID NO: 18: Amino acid sequence of β-chain CDR2 of pt. 2-ex08TCR
SEQ ID NO: 19: Amino acid sequence of α-chain CDR1 of pt. 2-ex08TCR
SEQ ID NO: 20: Amino acid sequence of α-chain CDR2 of pt. 2-ex08TCR
SEQ ID NO: 21: Amino acid sequence of α-chain CDR3 of pt. 2-ex08TCR
SEQ ID NO: 22: full-length amino acid sequence of β-chain of pt. 2-ex08TCR
SEQ ID NO: 23: full-length amino acid sequence of α-chain of pt. 2-ex08TCR
SEQ ID NO: 24: Amino acid sequence of β-chain CDR1 of pt. 1-ex08TCR
SEQ ID NO: 25: Amino acid sequence of β-chain CDR2 of pt. 1-ex08TCR
SEQ ID NO: 26: Amino acid sequence of α-chain CDR1 of pt. 1-ex08TCR
SEQ ID NO: 27: Amino acid sequence of α-chain CDR2 of pt. 1-ex08TCR
SEQ ID NO: 28: Amino acid sequence of α-chain CDR3 of pt. 1-ex08TCR
SEQ ID NO: 29: Full length amino acid sequence of β-chain of pt. 1-ex08TCR
SEQ ID NO: 30: full-length amino acid sequence of α-chain of pt. 1-ex08TCR
SEQ ID NO: 31: Amino acid sequence of β-chain CDR1 of pt. 1-ex15TCR
SEQ ID NO: 32: Amino acid sequence of β-chain CDR2 of pt. 1-ex15TCR
SEQ ID NO: 33: Amino acid sequence of α-chain CDR1 of pt. 1-ex15TCR
SEQ ID NO: 34: Amino acid sequence of α-chain CDR2 of pt. 1-ex15TCR
SEQ ID NO: 35: Amino acid sequence of α-chain CDR3 of pt. 1-ex15TCR
SEQ ID NO: 36: full-length amino acid sequence of β-chain of pt. 1-ex15TCR
SEQ ID NO: 37: full-length amino acid sequence of α-chain of pt. 1-ex15TCR
SEQ ID NO: 38: Amino acid sequence of β-chain CDR1 of pt. 2-ex09TCR
SEQ ID NO: 39: Amino acid sequence of β-chain CDR2 of pt. 2-ex09TCR
SEQ ID NO: 40: Amino acid sequence of α-chain CDR1 of pt. 2-ex09TCR
SEQ ID NO: 41: Amino acid sequence of α-chain CDR2 of pt. 2-ex09TCR
SEQ ID NO: 42: Amino acid sequence of α-chain CDR3 of pt. 2-ex09TCR
SEQ ID NO: 43: full-length amino acid sequence of β-chain of pt. 2-ex09TCR
SEQ ID NO: 44: full-length amino acid sequence of α-chain of pt. 2-ex09TCR

## Claims

1. A pharmaceutical composition for administering a peptide, a polynucleotide encoding the peptide, or a vector containing the polynucleotide, in combination with
a T cell,
for treating or preventing cancer expressing KitaKyushu Lung Cancer Antigen 1 (KK-LC-1) in a subject, wherein
the peptide and the T cell have the following combination (1) or (2) depending on a human leukocyte antigen (HLA) type that the subject has:
(1) a combination of a peptide comprising an amino acid sequence of SEQ ID NO: 1 and a T cell expressing a T cell receptor (TCR) having a β-chain comprising complementarity determining region 3 (CDR3) of SEQ ID NO: 2, when the HLA type of the subject is HLA*C14,
(2) a combination of a peptide comprising an amino acid sequence of SEQ ID NO: 3 and a T cell expressing a TCR having a β-chain comprising CDR3 of SEQ ID NO: 4, 5 or 6 when the HLA type of the subject is HLA*B15.

2. A pharmaceutical composition for administering a peptide, a polynucleotide encoding the peptide, or a vector containing the polynucleotide, in combination with
a T cell,
for treating or preventing cancer expressing KK-LC-1 in a subject, wherein
the peptide and the T cell have a combination of the following (1) or (2):
(1) a combination of a peptide comprising an amino acid sequence of SEQ ID **NO:** 1 and a T cell expressing a TCR having a β-chain comprising CDR3 of SEQ ID **NO:** 2,
(2) a combination of a peptide comprising an amino acid sequence of SEQ ID **NO:** 3 and a T cell expressing a TCR having a β-chain comprising CDR3 of SEQ ID **NO:** 4, 5 or **6.**

3. The pharmaceutical composition according to claim 2, wherein
an HLA type that the subject has is
HLA*C14 in the combination (1), and
HLA*B15 in the combination (2).

4. The pharmaceutical composition according to claim 3, wherein
the HLA type that the subject has is
HLA*C14:02 in the combination (1), and
HLA*B15:01 in the combination (2).

5. The pharmaceutical composition according to any one of claims 1 to 4, wherein the cancer is lung cancer, stomach cancer, breast cancer, or liver cancer.

6. A pharmaceutical composition comprising a peptide, a polynucleotide encoding the peptide or a vector containing the polynucleotide, which is administered in combination with a T cell for treating or preventing cancer expressing KK-LC-1 in a subject, wherein
the peptide is the following (1) or (2):
(1) a peptide comprising an amino acid sequence of SEQ ID NO: 1, which is administered in combination with a T cell expressing a TCR having a β-chain comprising CDR3 of SEQ ID NO: 2,
(2) a peptide comprising an amino acid sequence of SEQ ID NO: 3, which is administered in combination with a T cell expressing a TCR having a β-chain comprising CDR3 of SEQ ID NO: 4, 5 or 6.

7. A pharmaceutical composition for treating or preventing cancer expressing KK-LC-1 in a subject, comprising a peptide, a polynucleotide encoding the peptide, or a vector containing the polynucleotide, wherein
the peptide is the following (1) or (2):
(1) a peptide comprising an amino acid sequence of SEQ ID NO: 1,
(2) a peptide comprising an amino acid sequence of SEQ ID NO: 3.

8. The pharmaceutical composition according to claim 6 or 7, which is a cancer vaccine.

9. The pharmaceutical composition according to any one of claims 6 to 8, wherein the cancer is lung cancer, stomach cancer, breast cancer, or liver cancer.

10. A peptide comprising an amino acid sequence of SEQ ID NO: 1 or 3, a polynucleotide encoding the peptide, or a vector comprising the polynucleotide.

11. A pharmaceutical composition comprising a T cell, which is administered in combination with a peptide, a polynucleotide encoding the peptide, or a vector containing the polynucleotide, for treating or preventing cancer expressing KK-LC-1 in a subject, wherein
the T cell is the following (1) or (2):
(1) a T cell expressing a TCR having a β-chain comprising CDR3 of SEQ ID NO: 2, which is administered in combination with a peptide comprising an amino acid sequence of SEQ ID NO: 1,
(2) a T cell expressing a TCR having a β-chain comprising CDR3 of SEQ ID NO: 4, 5 or 6, which is administered in combination with a peptide comprising an amino acid sequence of SEQ ID NO: 3.

12. A pharmaceutical composition for treating or preventing cancer expressing KK-LC-1 in a subject, comprising a T cell, wherein
the T cell is the following (1) or (2):
(1) a T cell expressing a TCR having a β-chain comprising CDR3 of SEQ ID NO: 2,
(2) a T cell expressing a TCR having a β-chain comprising CDR3 of SEQ ID NO: 4, 5 or 6.

13. A polynucleotide encoding a TCR having a β-chain comprising CDR3 of SEQ ID NO: 2, 4, 5 or 6, or a vector comprising the polynucleotide.

14. A method for treating or preventing cancer expressing KK-LC-1 in a subject, comprising
a step of administering
a peptide, a polynucleotide encoding the peptide, or a vector containing the polynucleotide, in combination with
a T cell,
to the subject, wherein
the peptide and the T cell have a combination of the following (1) or (2) depending on a human leukocyte antigen (HLA) type that the subject has
(1) a combination of a peptide comprising an amino acid sequence of SEQ ID NO: 1 and a T cell expressing a TCR having a β-chain comprising CDR3 of SEQ ID NO: 2,
(2) a combination of a peptide comprising an amino acid sequence of SEQ ID NO: 3 and a T cell expressing a TCR having a β-chain comprising CDR3 of SEQ ID NO: 4, 5 or 6.

15. A method for treating or preventing cancer expressing KK-LC-1 in a subject, comprising
a step of determining an HLA type of the subject, and
a step of administering a peptide, a polynucleotide encoding the peptide, or a vector containing the polynucleotide in combination with
a T cell, wherein
(1) administering a peptide comprising an amino acid sequence of SEQ ID NO: 1 in combination with a T cell expressing a TCR having a β-chain comprising CDR3 of SEQ ID NO: 2, when the HLA type of the subject is HLA*C14, and
(2) administering a peptide comprising an amino acid sequence of SEQ ID NO: 3 in combination with a T cell expressing a TCR having a β-chain comprising CDR3 of SEQ ID NO: 4, 5 or 6, when the HLA type of the subject is HLA*B15.

16. A method for treating or preventing cancer expressing KK-LC-1 in a subject, comprising
a step of administering a peptide, a polynucleotide encoding the peptide, or a vector containing the polynucleotide to the subject, wherein
the peptide is the following (1) or (2):
(1) a peptide comprising an amino acid sequence of SEQ ID NO: 1,
(2) a peptide comprising an amino acid sequence of SEQ ID NO: 3.

17. A method for treating or preventing cancer expressing KK-LC-1 in a subject, comprising:
a step of determining an HLA type of the subject, and
a step of administering a peptide, a polynucleotide encoding the peptide, or a vector containing the polynucleotide to the subject, wherein
the peptide is the following (1) or (2):
(1) a peptide comprising an amino acid sequence of SEQ ID NO: 1, when the HLA type of the subject is HLA*C14,
(2) a peptide comprising the amino acid sequence of SEQ ID NO: 3, when the HLA type of the subject is HLA*B15.

18. A method for treating or preventing cancer expressing KK-LC-1 in a subject, comprising:
a step of administering a T cell to the subject,
wherein the T cell is the following (1) or (2):
(1) a T cell expressing a TCR having a β-chain comprising CDR3 of SEQ ID NO: 2,
(2) a T cell expressing a TCR having a β-chain comprising CDR3 of SEQ ID NO: 4, 5 or 6.

19. A method for treating or preventing cancer expressing KK-LC-1 in a subject, comprising
a step of determining an HLA type of the subject, and
a step of administering a T cell to the subject,
wherein the T cell is the following (1) or (2):
(1) a T cell expressing a TCR having a β-chain comprising CDR3 of SEQ ID NO: 2, when the HLA type of the subject is HLA*C14,
(2) a T cell expressing a TCR having a β-chain comprising CDR3 of SEQ ID NO: 4, 5 or 6, when the HLA type of the subject is HLA*B15.

20. Use of a peptide, a polynucleotide encoding the peptide, or a vector containing the polynucleotide, or
a T cell,
for producing a pharmaceutical composition for treating or preventing cancer expressing KK-LC-1 in a subject, wherein
the pharmaceutical composition has a combination of the peptide of the following (1) or (2) and the T cell:
(1) a combination of a peptide comprising an amino acid sequence of SEQ ID NO: 1 and a T cell expressing a T cell receptor (TCR) having a β-chain comprising CDR3 of SEQ ID NO: 2,
(2) a combination of a peptide comprising an amino acid sequence of SEQ ID NO: 3 and a T cell expressing a TCR having a β-chain comprising CDR3 of SEQ ID NO: 4, 5 or 6.
